# DEMANDE DE BREVET EUROPEEN

(11) **EP 1 454 612 A1**
(43) Date de publication de la demande: **08.09.2004**
(21) Numéro de dépôt: 04290612.3
(22) Date de dépôt: 05.03.2004
(51) Int. Cl.: A61K 7/02, A61K 7/48, A61K 7/04, A61K 7/025, A61K 7/07

(54) **Composition cosmétique contenant un ester et un composé pâteux**

(30) Priorité: 06.03.2003 FR 0302809; 06.03.2003 FR 0302811
(71) Demandeur: L'OREAL, 75008 Paris (FR)
(72) Inventeur: Filippi, Vanina, 75015 Paris (FR); Le Chaux, Laure, 94550 Chevilly-Larue (FR)
(74) Mandataire: Le Coupanec, Pascale

(57) **Abrégé**

La présente invention concerne une composition cosmétique de maquillage et/ou de soin de la peau, des lèvres et/ou des phanères comprenant dans un milieu physiologiquement acceptable au moins un ester de dimère diol et d'au moins un acide mono-carboxylique en C₄ à C₃₄ ou di-carboxylique, au moins un composé pâteux et au moins un agent de coloration, ladite composition possédant une brillance moyenne supérieure ou égale à 30.

## Description

La présente invention se rapporte à une composition cosmétique de maquillage ou de soin de la peau, y compris du cuir chevelu, aussi bien du visage que du corps humain, des lèvres ou des phanères des êtres humains, comme les cheveux, les cils, les sourcils ou les ongles, comprenant un milieu cosmétiquement acceptable.

La composition de l'invention peut en particulier constituer un produit de maquillage du corps, des lèvres ou des phanères d'êtres humains ayant en particulier des propriétés de soin et/ou de traitement non thérapeutique. Elle constitue notamment un rouge à lèvres ou un brillant à lèvres, un fard à joues ou à paupières, un produit pour tatouage, un mascara, un eye-liner, un vernis à ongles, un produit de bronzage artificiel de la peau, un produit de coloration ou de soin des cheveux.

Il existe de nombreuses compositions cosmétiques pour lesquelles les propriétés de brillance du film déposé, après application sur les matières kératiniques (peau, lèvres, phanères), sont souhaitables. On peut citer par exemple les rouges à lèvres, les vernis à ongles ou encore certains produits capillaires. Pour ce faire, le formulateur utilise généralement à titre d'agent actif principal en terme de brillance des lanolines associées à au moins une huile dite « brillante », comme des polymères huileux tels que a) les polybutènes qui ont une viscosité élevée, b) des esters d'acide ou d'alcool gras dont le nombre de carbone est élevé (typiquement supérieur à 16), ou encore c) certaines huiles végétales.

Toutefois, les polymères huileux comme les polybutènes présentent l'inconvénient d'être très collants à l'application et dans le temps ce qui est rédhibitoire en terme de confort pour l'utilisateur.

L'invention a précisément pour objet de proposer une association particulière comprenant au moins un ester de dimère diol avec un acide mono-carboxylique ou dicarboxylique et en particulier un diacide dicarboxylique spécifique permettant avantageusement de donner satisfaction en terme de brillance sans par ailleurs affecter l'aspect confort de la composition cosmétique incorporant ladite association.

Les compositions ainsi obtenues ne posent pas de problème particulier de collant et sont dénuées d'odeur désagréable.

Ainsi, les lanolines présentent l'inconvénient d'être sensibles à la chaleur et aux ultraviolets et ont tendance à s'oxyder dans le temps avec un dégagement d'odeur désagréable. De plus, lorsque les lanolines sont associées avec des huiles couramment utilisées dans le domaine cosmétique, les compositions obtenues présentent des problèmes de collant qui sont d'autant plus prononcés que l'huile utilisée possède une viscosité élevée.

Ce type d'association est d'autant plus avantageux qu'il permet de s'affranchir de l'utilisation de composés usuels comme la lanoline et la vaseline et qui ne sont plus, dans certaines circonstances, jugés souhaitables, notamment pour une raison de défaut de stabilité, dans les formulations cosmétiques.

Les esters de dimères diols et d'acides mono ou dicarboxyliques ont été décrits d'une manière générale dans le document FR 2 795 309 comme étant utiles pour préparer des compositions cosmétiques dotées notamment de propriétés de stabilité améliorées. Plus récemment, les documents JP 2002-128623, 2002-128628 et 2002-128629 proposent des compositions cosmétiques, notamment de maquillage, incluant à titre d'agent actif pour la brillance des esters de diacides dilinoléiques avec des dimères diols dilinoléiques.

Plus précisément, la présente invention concerne, selon un de ses aspects, une composition cosmétique de maquillage et/ou de soin de la peau, des lèvres et/ou des phanères comprenant dans un milieu physiologiquement acceptable au moins un ester de dimère diol et d'au moins un acide mono-carboxylique en C₄-C₃₄ ou dicarboxylique, au moins un composé pâteux et au moins un agent de coloration, ladite composition possédant une brillance moyenne supérieure ou égale à 30.

La présente invention concerne, selon un autre de ses aspects, une composition cosmétique anhydre de maquillage et/ou de soin de la peau, des lèvres et/ou des phanères comprenant dans un milieu physiologiquement acceptable au moins un ester de dimère diol et d'acide gras ou de dimère diacide d'acide gras, au moins un composé pâteux et moins de 15% en poids d'au moins une charge.

La présente invention concerne, en outre, une composition cosmétique de maquillage et/ou de soin de la peau, des lèvres et/ou des phanères comprenant dans un milieu physiologiquement acceptable au moins un ester de dimère diol et de dimère diacide d'acides gras insaturés et au moins un composé pâteux.

La présente invention concerne, aussi, un procédé de maquillage et/ou de soin de la peau, des lèvres et/ou des phanères comprenant l'application sur la peau, les lèvres et/ou les phanères d'au moins une composition selon l'invention.

### BRILLANCE

D'une manière générale, les compositions cosmétiques et plus particulièrement celles destinées à être appliquées sur les lèvres peuvent être caractérisées par un indice de brillance moyenne.

Par «brillance moyenne », on entend la brillance telle qu'elle peut être mesurée à l'aide d'un brillancemètre, de manière conventionnelle, par la méthode suivante :

Sur une carte de contraste de marque LENETA® et de référence FORM 1A PENOPAC, on étale une couche de 50 µm d'épaisseur de la composition dont on cherche à évaluer la brillance moyenne, à l'aide d'un étaleur automatique. La couche recouvre au moins le fond blanc de la carte. On procède de suite à la mesure de la brillance à 20° sur le fond blanc à l'aide d'un brillancemètre de marque BYK GARDNER® et de référence microTRI-GLOSS.

La brillance moyenne de la composition est avantageusement supérieure ou égale à 30, voire supérieure ou égale à 40, notamment supérieure ou égale à 50, en particulier supérieure ou égale à 60, plus particulièrement supérieure ou égale à 65 ou mieux supérieure ou égale à 70, notamment lorsque la composition est destinée à être appliquée sur les lèvres.

Par exemple, une composition cosmétique de type rouge à lèvres peut posséder une brillance moyenne égale à 60 environ, une composition cosmétique de type base de gloss liquide ou une composition cosmétique de type fard à paupières de brillance moyenne égale à 70 environ, et une composition cosmétique de type base de vernis à ongles de brillance moyenne égale à 50 environ.

On désigne par « gloss liquide », encore appelé rouge à lèvres liquide ou brillant à lèvres, un produit fluide destiné à être appliqué sur les lèvres et conditionné par exemple dans un récipient pourvu d'un applicateur, cet applicateur comportant un organe de préhension qui sert également de capuchon de fermeture du récipient, et un élément d'application.

Selon une variante de l'invention, la composition cosmétique est sous forme solide ou encore coulée. Au sens de la présente invention, on entend qualifier par « solide », toute composition cosmétique dénuée de faculté d'écoulement sous l'action de son propre poids.

Ces compositions peuvent, le cas échéant, présenter un aspect pâteux à température ambiante (25 °C). Ainsi une composition cosmétique pourra être conforme à l'invention à partir du moment où elle possédera un point de fusion ou une température de transition thermique (par exemple un point de ramollissement), supérieur à 25 °C, notamment pouvant varier de 25 à 85 °C, voire de 30 à 60 °C, et en particulier de 30 à 45 °C et/ou une dureté pouvant varier de 0,001 à 0,5MPa, notamment de 0,005 à 0,4MPa.

La dureté d'une composition peut être déterminée par la mesure de la force en compression mesurée à 20 °C à l'aide du texturomètre vendu sous la dénomination TA-XT2i® par la société RHEO, équipé d'un cylindre en inox d'un diamètre de 2 mm se déplaçant à la vitesse de mesure de 0,1 mm/s, et pénétrant dans la composition à une profondeur de pénétration de 0,3 mm. La valeur de la dureté est la force de compression maximale mesurée divisée par la surface du cylindre du texturomètre en contact avec la composition. Dans le cas particulier des rouges à lèvres, la dureté peut également être mesurée par la méthode dite du fil à couper le beurre, qui consiste à couper un bâton de rouge à lèvres de 8,1 mm de diamètre et à mesurer la dureté à 20 °C, au moyen d'un dynamomètre DFGHS 2® de la société Indelco-Chatillon se déplaçant à une vitesse de 100 mm/minute. La dureté mesurée est exprimée comme la force de cisaillement (exprimée en grammeforce) nécessaire pour couper un stick dans ces conditions. Selon cette méthode la dureté d'une composition en stick selon l'invention va notamment de 50 à 300 g, de préférence de 100 à 250 g et par exemple de 150 à 230 g. Les valeurs les plus élevées étant généralement privilégiées pour les compositions destinées à être formulées en bâton ayant un diamètre de 12,7 mm et les valeurs les plus basses pour celles destinées à des bâtons de 8,3 mm de diamètre.

### ESTER DE DIMÈRE DIOL ET DE DIMERE DIACIDE D'ACIDES GRAS INSATURES

Les esters de dimère diol utilisables dans le cadre de la présente invention sont disponibles commercialement ou peuvent être préparés de manière conventionnelle. Ils sont notamment d'origine végétale et peuvent être obtenus par estérification d'un dimère diol, avec un acide mono-carboxylique en C₄-C₃₄ comme par exemple un acide gras ou avec un acide dicarboxylique tel qu'un dimère diacide.

Dans le cas de l'estérification avec un acide mono-carboxylique, on peut obtenir des esters ayant un poids moléculaire relativement élevé, allant d'environ 1000 à 1300 g/mol, on peut obtenir un dicarboxylate de dimère diol qui présente un poids moléculaire moyen en poids, déterminé par chromatographie de perméation de gel (GPC), allant de 2000 à 20 000 g/mol, de préférence entre 2000 et 4000 g/mol.

### Acide mono-carboxylique en C₄-C₃₄

L'acide mono-carboxylique utilisable dans la présente invention comporte de 4 à 34 atomes de carbone, et notamment de 10 à 32 atomes de carbone.

A titre illustratif des exemples d'acide mono-carboxylique convenant à l'invention, on peut notamment citer :
- les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide heptadécanoïque, l'acide hexadécanoïque, l'acide pentadécanoïque, l'acide octadécanoïque, l'acide nonadécanoïque, l'acide eicosanoïque, l'acide docosanoïque, l'acide tétracosanoïque,
- les acides gras ramifiés tels que par exemple l'acide isobutanoïque, l'acide isopentanoïque, l'acide pivalique, l'acide isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque, des acides gras à longue chaîne obtenus à partir de la lanoline,
- les acides gras linéaires insaturés en C₈ à C₃₄, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique,
- des hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque,
- des acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique, et
- des acides gras d'origine naturelle, tels que les acides gras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison et leurs mélanges.

Il s'agit plus particulièrement d'un acide gras, notamment tel que défini ci-dessus.

L'ester obtenu peut être un diester, un monoester ou un de leurs mélanges. En l'occurrence, l'ester peut être un mélange de deux ou plusieurs types d'ester formés avec différents acides carboxyliques.

### Acide dicarboxylique

L'acide dicarboxylique utilisable selon l'invention doit contenir au moins deux groupes carboxyliques par molécule.

Il peut notamment être représenté par la formule (I) suivante :

HOOC-(CH₂)ₙ-COOH (I)

dans laquelle n est un nombre entier de 1 à 16, de préférence de 3 à 16.

A titre illustratif et non limitatif des acides dicarboxyliques convenant à l'invention, on peut notamment citer l'acide malonique, l'acide succinique, l'acide glutarique, l'acide adipique, l'acide pimélique, l'acide subérique, l'acide azélaïque, l'acide sébacique, l'acide 1,9-nonaméthylène-dicarboxylique, l'acide 1,10-décaméthylènedicarboxylique, l'acide 1,11-undécaméthylènedicarboxylique, l'acide 1,12-dodécaméthylène-dicarboxylique, l'acide 1,13-tridécaméthylènedicarboxylique, l'acide 1,14-tétradécaméthylènedicarboxylique, l'acide 1,15-pentadécaméthylènedicarboxylique, l'acide 1,16-hexadécaméthylènedicarboxylique et leurs mélanges.

L'acide dicarboxylique peut également être un dimère diacide. Un dimère diacide désigne un diacide obtenu par réaction de polymérisation, notamment de dimérisation, intermoléculaire d'au moins un acide gras insaturé.

Ils dérivent en particulier de la dimérisation d'un acide gras insaturé notamment en C₈ à C₃₄, notamment en C₁₂ à C₂₂, en particulier en C₁₆ à C₂₀, et plus particulièrement en C₁₈.

A titre représentatif de ces acides gras insaturés, on peut notamment citer comme précédemment l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

Selon une variante particulière, il s'agit plus particulièrement du dimère diacide dont dérive également le dimère diol à estérifier.

Plus particulièrement, il s'agit du dimère diacide obtenu par dimérisation de l'acide linoléique et notamment de sa forme hydrogénée. Cette forme hydrogénée peut être partielle ou totale et notamment correspondre à la forme saturée plus stable à l'oxydation.

Selon un mode de réalisation, le dimère diacide est un produit commercialisé constitué d'un acide dicarboxylique ayant environ 36 atomes de carbone. Ce produit contient également un acide trimérique et un acide monomère, dans des proportions qui dépendent du degré de pureté du produit. Classiquement, on trouve dans le commerce des produits dont la teneur en dimère diacide est supérieure à 70 % et d'autres dont la teneur en dimère diacide a été ajustée à 90 % ou plus.

On trouve également dans le commerce des dimères diacides et notamment des diacides dilinoléiques dont la stabilité vis-à-vis de l'oxydation a été améliorée par hydrogénation des doubles liaisons restant après la réaction de dimérisation.

Dans la présente invention, on peut utiliser tout dimère diacide disponible actuellement dans le commerce.

Dans une réaction d'estérification avec un acide dicarboxylique et en particulier un dimère diacide, le degré moyen d'estérification et le poids moléculaire moyen de l'ester obtenu peuvent être ajustés en faisant varier le rapport du dimère diol à l'acide dicarboxylique et en particulier au dimère diacide.

Le rapport, exprimé comme la proportion molaire d'un acide dicarboxylique basée sur le poids moléculaire moyen calculé à partir de son indice d'acide pour 1 mole de dimère diol basée sur le poids moléculaire moyen calculé à partir de son indice d'hydroxyle, est compris généralement entre 0,2 et 1,2 mole, en particulier entre 0,4 et 1,0, par exemple égal à 0,5 ou 0,7.

### Dimère diol

Au sens de la présente invention, on entend plus particulièrement désigner sous le terme « dimère diol » des diols saturés produits par hydrogénation des dimères diacides correspondants, un dimère diacide étant tel que défini ci-dessus.

En ce qui concerne le dimère diol fabriqué industriellement, il contient également généralement d'autres composants, par exemple, un trimère triol, un monoalcool et des composés de type éther, selon le degré de purification de l'acide dimérique et/ou de l'ester d'alcool inférieur de celui-ci, utilisé comme matière première. Généralement, les produits dont la teneur en dimère diol est supérieure à 70 % peuvent être utilisés dans la présente invention. Toutefois, il est préférable d'utiliser un dimère diol de grande pureté, tel qu'un composé dont la teneur en dimère diol est supérieure à 90 %.

Ainsi, un dimère diol peut être produit par hydrogénation catalytique d'un dimère diacide, lui-même obtenu par dimérisation d'au moins un acide gras insaturé notamment en C₈ à C₃₄, tels que ceux cités précédemment, notamment en C₁₂ à C₂₂, en particulier en C₁₆ à C₂₀, et plus particulièrement en C₁₈ à l'image par exemple de l'acide oléique et de l'acide linoléique.

Selon un mode de réalisation particulier, le dimère diol dérive de l'hydrogénation du diacide dilinoléique. Il est généralement sous une forme saturée.

Selon une variante de l'invention, l'ester de dimère diol est un ester de dimère diol et de dimère diacide et notamment est un composé de formule générale (II)

HO-R¹-(-OCO-R²-COO-R¹-)ₕ-OH (II)

dans laquelle :
R¹ représente un reste de dimère diol obtenu par hydrogénation du diacide dilinoléique
R² représente un reste de diacide dilinoléique hydrogéné, et
h représente un entier variant de 1 à 9.

A titre illustratif des esters convenant à l'invention, on peut notamment citer les esters de diacides dilinoléiques et de dimères diols dilinoléiques commercialisés par la société NIPPON FINE CHEMICAL sous la dénomination commerciale LUSPLAN DD-DA5® et DD-DA7® .

La quantité en ester selon l'invention est généralement ajustée de manière à contrôler la brillance moyenne de la composition à la valeur souhaitée. En l'occurrence, l'ester peut être présent à raison de 1 à 99 % en particulier, notamment de 2 à 60 % en poids, en particulier de 5 à 40 % et plus particulièrement de 10 à 35 % en poids par rapport au poids total de la composition.

### COMPOSE PATEUX

Par "pâteux" au sens de la présente invention, on entend désigner un composé gras lipophile, autre que la lanoline et ses dérivés, à changement d'état solide/liquide réversible présentant à l'état solide une organisation cristalline anisotrope, et comportant à la température de 23°C une fraction liquide et une fraction solide. A titre illustratif des dérivés de lanoline exclus des composés pâteux selon l'invention, on peut notamment citer la lanoline liquide, la lanoline réduite, la lanoline purifiée par adsorption, l'acétate de lanoline, la cire de lanoline, l'acétate de lanoline liquide, l'hydroxylanoline, la polyoxyéthylène-lanoline, l'acide gras de lanoline, l'acide gras de lanoline dure, les esters de cholestéryle d'acide gras de lanoline, l'alcool de lanoline, l'acétate de l'alcool de lanoline, et autres.

Par composé pâteux au sens de l'invention, on entend un composé ayant une dureté à 20°C allant de 0,001 à 0,5 MPa, de préférence de 0,002 à 0,4 MPa. La dureté est mesurée selon le protocole décrit précédemment à l'aide d'un analyseur de texture, par exemple le TA-XT2i® de chez Rhéo.

Ce composé pâteux est en outre, à la température de 23 °C, sous la forme d'une fraction liquide et d'une fraction solide. En d'autres termes, la température de fusion commençante du composé pâteux est inférieure à 23°C. La fraction liquide du composé pâteux mesurée à 23 °C représente 9 à 97% en poids du composé. Cette fraction liquide à 23 °C représente de préférence entre 15 et 85%, de préférence encore entre 40 et 85% en poids.

La fraction liquide en poids du composé pâteux à 23°C est égale au rapport de l'enthalpie de fusion consommée à 23°C sur l'enthalpie de fusion du composé pâteux.

L'enthalpie de fusion du composé pâteux est l'enthalpie consommée par le composé pour passer de l'état solide à l'état liquide. Le composé pâteux est dit à l'état solide lorsque l'intégralité de sa masse est sous forme solide cristalline. Le composé pâteux est dit à l'état liquide lorsque l'intégralité de sa masse est sous forme liquide.

L'enthalpie de fusion du composé pâteux est égale à l'aire sous la courbe du thermogramme obtenu à l'aide d'un calorimètre à balayage différentiel (D. S. C), tel que le calorimètre vendu sous la dénomination MDSC 2920® par la société TA instrument, avec une montée en température de 5 ou 10°C par minute, selon la norme ISO 11357-3:1999. L'enthalpie de fusion du composé pâteux est la quantité d'énergie nécessaire pour faire passer le composé de l'état solide à l'état liquide. Elle est exprimée eu J/g.

L'enthalpie de fusion consommée à 23°C est la quantité d'énergie absorbée par l'échantillon pour passer de l'état solide à l'état qu'il présente à 23°C constitué d'une fraction liquide et d'une fraction solide.

La fraction liquide du composé pâteux mesurée à 32°C représente de préférence de 30 à 100% en poids du composé, de préférence de 80 à 100%, de préférence encore de 90 à 100% en poids du composé. Lorsque la fraction liquide du composé pâteux mesurée à 32°C est égale à 100%, la température de la fin de la plage de fusion du composé pâteux est inférieure ou égale à 32°C.

La fraction liquide du composé pâteux mesurée à 32°C est égale au rapport de l'enthalpie de fusion consommée à 32°C sur l'enthalpie de fusion du composé pâteux. L'enthalpie de fusion consommée à 32°C est calculée de la même façon que l'enthalpie de fusion consommée à 23°C.

Le composé pâteux est de préférence choisi parmi les composés synthétiques et les composés d'origine végétale. Un composé pâteux peut être obtenu par synthèse à partir de produits de départ d'origine végétale.

Le composé pâteux est avantageusement choisi parmi :
- les composés siliconés polymères ou non,
- les composés fluorés polymères ou non,
- les polymères vinyliques, notamment :

- les homopolymères d'oléfines
- les copolymères d'oléfines
- les homopolymères et copolymères de diènes hydrogénés
- les oligomères linéaires ou ramifiés, homo ou copolymères de (méth)acrylates d'alkyles ayant de préférence un groupement alkyle en C₈-C₃₀
- les oligomères homo et copolymères d'esters vinyliques ayant des groupements alkyles en C₈-C₃₀
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
et leurs mélanges.

Le composé pâteux est de préférence un polymère, notamment hydrocarboné.

### Composés pâteux siliconés et /ou fluorés

Un composé pâteux siliconé et fluoré préféré est le polyméthyl trifluoropropryl méthylalkyl diméthylsiloxane, fabriqué sous la dénomination X22-1088® par SHIN ETSU.

Lorsque le composé pâteux est un polymère siliconé et/ou fluoré, la composition comprend avantageusement un agent compatibilisant tel que les esters à courte chaîne comme le néopentanoate d'isodécyle.

### Composés pâteux polyéthers

Parmi les polyéthers liposolubles, on préfère en particulier les copolymères d'éthylène-oxyde et/ou de propylène-oxyde avec des alkylènes-oxydes à longue chaîne en C₆-C₃₀, de préférence encore tels que le rapport pondéral de l'éthylène-oxyde et/ou de propylène-oxyde avec alkylènes-oxydes dans le copolymère est de 5:95 à 70:30. Dans cette famille, on citera notamment les copolymères tels que les alkylènes-oxydes à longue chaîne sont disposés en blocs ayant un poids moléculaire moyen de 1.000 à 10.000, par exemple un copolymère bloc de polyoxyéthylène/polydodécyle glycol tel que les éthers de dodécanediol (22 mol) et de polyéthylène glycol (45 OE) commercialisés sous la marque ELFACOS ST9® par Akzo Nobel.

Parmi les esters, on préfère notamment :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique, à l'image notamment de ceux commercialisé sous la marque Softisan 649® par la société Sasol,
- le propionate d'arachidyle commercialisé sous la marque Waxenol 801® par Alzo,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique; et leurs mélanges.

L'acide carboxylique aliphatique comprend de 4 à 30 et de préférence de 8 à 30 atomes de carbone. Il est de préférence choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

L'acide carboxylique aliphatique est de préférence ramifié.

L'ester d'acide hydroxy carboxylique aliphatique est avantageusement issu d'un acide carboxylique aliphatique hydroxylé comportant de 2 à 40 atomes de carbone, de préférence de 10 à 34 atomes de carbone et mieux de 12 à 28 atomes de carbone, et de 1 à 20 groupes hydroxyle, de préférence de 1 à 10 groupes hydroxyle et mieux de 1 à 6 groupes hydroxyle. L'ester d'acide hydroxy carboxylique aliphatique est choisi parmi :
a) les esters partiels ou totaux de monoacides carboxyliques aliphatiques mono hydroxylés linéaires, saturés;
b) les esters partiels ou totaux de monoacides carboxyliques aliphatiques mono hydroxylés insaturés ;
c) les esters partiels ou totaux de polyacides carboxyliques aliphatiques mono hydroxylés saturés ;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé, et leurs mélanges.

Les esters aliphatiques d'ester sont avantageusement choisis parmi :
- l'ester résultant de la réaction d'estérification de l'huile de ricin hydrogénée avec l'acide isostéarique dans les proportions 1 pour 1 (1/1) ou monoisostéarate d'huile de ricin hydrogénée,
- l'ester résultant de la réaction d'estérification de l'huile de ricin hydrogénée avec l'acide isostéarique dans les proportions 1 pour 2 (1/2) ou le diisostéarate d'huile de ricin hydrogénée,
- l'ester résultant de la réaction d'estérification de l'huile de ricin hydrogénée avec l'acide isostéarique dans les proportions 1 pour 3 (1/3) ou triisostéarate d'huile de ricin hydrogénée,
- et leurs mélanges.

Parmi les composés pâteux d'origine végétale, on choisira de préférence un mélange de stérols de soja et de pentaérythritol oxyéthyléné (5OE) oxypropyléné (5 OP), commercialisé sous la référence Lanolide® par la société VEVY.

Le composé pâteux représente de préférence 1 à 99%, mieux 1 à 60%, mieux 2 à 30% et mieux encore 5 à 15% en poids de la composition.

En particulier, le composé pâteux et l'ester peuvent être associés dans la composition cosmétique dans un rapport pondéral composé pâteux/ester variant de 0,25 à 0,75 , notamment de 0, 3 à 0,6.

### MILIEU PHYSIOLOGIQUEMENT ACCEPTABLE

Par « milieu physiologiquement acceptable », on désigne un milieu non toxique et susceptible d'être appliqué sur la peau ou les lèvres d'êtres humains. Le milieu physiologiquement acceptable est généralement adapté à la nature du support sur lequel doit être appliquée la composition ainsi qu'à l'aspect sous lequel la composition est destinée à être conditionnée.

### Phase aqueuse

La composition selon l'invention peut comprendre au moins un milieu aqueux, constituant une phase aqueuse, qui peut former la phase continue de la composition.

La phase aqueuse peut être constituée essentiellement d'eau.

Elle peut également comprendre un mélange d'eau et de solvant organique miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, et les aldéhydes en C₂-C₄.

La phase aqueuse (eau et éventuellement le solvant organique miscible à l'eau) peut être présente, à une teneur allant de 1 % à 95 % en poids, notamment allant de 3 % à 80 % en poids, et en particulier allant de 5 % à 60 % en poids par rapport au poids total de la composition.

Cette phase aqueuse peut, le cas échéant, être épaissie, gélifiée ou structurée en y incorporant en outre un gélifiant aqueux traditionnel notamment d'origine minérale comme l'argile par exemple et/ou organique comme un polymère gélifiant aqueux.

Un tel milieu peut également comprendre au moins une huile volatile telle que définie ci-après.

### Phase grasse

La composition, notamment lorsqu'elle est destinée à être appliquée sur les lèvres, peut comporter au moins une phase grasse et notamment au moins un corps gras liquide à température ambiante (25°C) et à pression atmosphérique et/ou un corps gras solide à température ambiante et à pression atmosphérique tel que les cires, les gommes et leurs mélanges. La phase grasse peut en outre contenir des agents gélifiants et structurants d'huiles de nature organique et/ou des solvants organiques lipophiles.

Selon une variante, la composition cosmétique selon l'invention peut notamment posséder une phase grasse continue, pouvant contenir moins de 5 % d'eau, notamment moins de 1 % d'eau par rapport à son poids total et en particulier peut être sous forme anhydre.

Selon une variante particulière de l'invention, la composition cosmétique est exempte de paraffine, de vaseline et, comme précisé précédemment, de lanoline ou de l'un de ses dérivés.

La phase grasse de la composition selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par "huile non volatile", on entend une huile restant sur la peau à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par "huile hydrocarbonée", une huile contenant principalement des atomes d'hydrogène et de carbone et éventuellement des atomes d'oxygène, d'azote, de soufre et/ou de phosphore.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permetyls® , les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alcoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans la composition selon l'invention à une teneur allant de 0,1 % à 98 % en poids, notamment de 1 % à 65 % en poids, et en particulier de 2 % à 50 % en poids, par rapport au poids total de la composition.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810® , 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₃COOR₄ dans laquelle R₃ représente le reste d'un acide gras linéaire ou ramifié comportant de 1 à 40 atomes de carbone et R₄ représente une chaîne hydrocarbonée notamment ramifiée contenant de 1 à 40 atomes de carbone à condition que R₃ + R₄ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates et leurs mélanges.

Les huiles non volatiles peuvent être présentes dans la composition selon l'invention en une teneur allant de 0,01 à 90 % en poids, notamment de 0,1 % à 85% en poids, et en particulier de 1 % à 70 % en poids, par rapport au poids total de la composition.

Les huiles peuvent représenter de 0 à 99 % du poids total de la composition, en particulier de 0,01 à 99 %, plus particulièrement de 0,05 à 60 % et mieux de 1 à 35 %.

On utilise en particulier dans le cadre de la présente invention des huiles dont le poids moléculaire est compris entre 650 et 10.000 g/mol, notamment entre 750 et 7.500 g/mol.

Selon un mode de mise en oeuvre, la composition de l'invention comprend une phase huileuse comprenant au moins 70 % en poids d'une huile de masse molaire comprise entre 650 et 10.000 g/mol, de préférence entre 750 et 7.500 g/mol. La phase huileuse comprend avantageusement plus de 80 %, en particulier plus de 85 % en poids d'un huile de masse molaire comprise entre 650 et 10.000 g/mol, plus particulièrement entre 750 et 7.500 g/mol.

L'huile de masse molaire élevée est de préférence choisie parmi les polymères lipophiles :
- les esters d'acides gras linéaires ayant un nombre total de carbone allant de 35 à 70,
- les esters hydroxylés,
- les esters aromatiques,
- les esters d'alcool gras ou d'acides gras ramifiés en C₂₄-C₂₈,
- les huiles siliconées,
- les huiles d'origine végétale,
et leurs mélanges.

L'huile de masse molaire élevée est de préférence choisie parmi les polybutylènes, les polyisobutylènes hydrogénés, les polydécènes, les polydécènes hydrogénés, les copolymères de la vinylpyrrolidone tel que le copolymère de PVP/héxadécène, le tétrapélargonate de pentaérythrityle, le triisostéarate de polyglycérol-2, le trimellitate de tridécyle, le citrate de triisoarachidyle, le tétraisononanoate de pentaérythrityle, le triisostéarate de glycéryle, le tétraisostéarate de pentaérythrityle, le tri décyl-2 tétradécanoate de glycéryle, le tétra décyl-2 tétradécanoate de pentaérythrityle, les silicones phénylées, l'huile de sésame, et leurs mélanges.

Plus généralement, le corps gras liquide à température ambiante et à pression atmosphérique peut être présent à raison de 0,01 à 90 % en poids et notamment de 0,1 à 85 % en poids, par rapport au poids de la phase grasse.

La composition cosmétique peut comporter, notamment dans le cas où elle est destinée à être appliquée sur les lèvres, une phase huileuse ayant un indice de réfraction compris entre 1,47 et 1,51, ce qui peut permettre d'obtenir une brillance relativement élevée.

En ce qui concerne le corps gras solide à température ambiante et à pression atmosphérique, il peut être choisi parmi les cires, les gommes et leurs mélanges. Ce corps gras solide peut être présent à raison de 0,01 à 50 %, notamment de 0,1 à 40 % et en particulier de 0,2 à 30 % en poids, par rapport au poids total de la phase grasse.

### Cires et gommes

En particulier, la composition peut contenir au moins une cire.

Par "cire" au sens de la présente invention, on entend un composé gras lipophile, solide à température ambiante (25°C), à changement d'état solide/liquide réversible, ayant une température de fusion supérieure à 30°C pouvant aller jusqu'à 200°C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40°C et mieux supérieure à 45°C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40°C et mieux à 45°C, les cires de silicones comme les alkyl- ou alkoxydiméthicones ayant une chaîne alkyle ou alcoxy de 10 à 45 atomes de carbone, les esters de poly(di)méthylsiloxane solide à 40°C dont la chaîne ester comporte au moins 10 atomes de carbone ; et leurs mélanges.

Les gommes utilisables dans l'invention se présentent généralement sous forme solubilisée dans une huile, les polymères sont solides à température ambiante et les résines peuvent être liquides ou solides à température ambiante.

Par gomme on entend un corps gras qui se présente sous forme de polymère solide à température ambiante ayant un poids moléculaire moyen en poids de 50 000 à 1 000 000. La gomme est souvent vendue en dispersion dans un solvant organique, du type huile de silicone.

La nature et la quantité des gommes ou cires sont fonction des propriétés mécaniques et des textures recherchées. A titre indicatif, la cire peut représenter de 0,01 à 50 %, de préférence de 2 à 40 %, et mieux de 5 à 30 % du poids total de la composition.

En particulier, la cire peut être présente sous forme d'émulsion cire(s)-dans-eau.

La cire peut être présente dans la composition selon l'invention en une teneur allant de 0,01 % à 50 % en poids, en particulier de 0,1 % à 30 % en poids, et notamment de 0,2 % à 20 % en poids, par rapport au poids total de la composition.

### Charge

Au sens de l'invention, on entend désigner sous ce terme tout composé, organique et/ou inorganique introduit dans la composition cosmétique afin d'ajuster ses propriétés en terme de texture ou en d'autres termes pour contrôler ses propriétés rhéologiques. Les pigments et nacres sont notamment exclus de cette définition.

Selon une variante particulière de l'invention, les compositions cosmétiques comprennent moins de 15% en poids, en particulier moins de 10% en poids, notamment moins de 7% en poids de charge par rapport au poids total de la composition.

Il peut notamment s'agir de charges sphériques comme par exemple le talc, le stéarate de zinc, le mica, le kaolin, les poudres de polyamide (Nylon® ) (Orgasol® de chez Atochem), les poudres de polyéthylène, les poudres de polymères de tétrafluoroéthylène (Téflon® ), l'amidon, le nitrure de bore, des microsphères polymériques telles que celles de chlorure de polyvinylidène/acrylonitrile comme l'Expancel® (Nobel Industrie), de copolymères d'acide acrylique (Polytrap® de la société Dow Corning), les microbilles de résine de silicone (Tospearls® de Toshiba, par exemple), et les organopolysiloxanes élastomères.

### Agents tensioactifs

La composition selon l'invention peut en outre contenir des agents tensioactifs émulsionnants présents notamment en une proportion allant de 0,1 à 30 % en poids, et mieux de 5 % à 15 % en poids, par rapport au poids total de la composition.

Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges,
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

### Agent de coloration

La composition de l'invention, comprend généralement au moins un agent de coloration pouvant notamment être présent à raison de 0,01 % à 40 % en poids, notamment de 0,01 % à 30 % en poids et en particulier de 0,05 % à 25 % en poids, par rapport au poids total de la composition.

Ces ou cet agent de coloration peuvent être choisis parmi les pigments, les colorants hydrosolubles ou liposolubles, les nacres et leurs mélanges.

Par pigments, il faut comprendre des particules blanches ou colorées, minérales ou organiques, insolubles dans la phase hydrophile liquide, destinées à colorer et/ou opacifier la composition. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées.

Les pigments peuvent être présents dans la composition à raison de 0,01 % à 25 % en poids, en particulier de 0,01 % à 15 % en poids, et notamment de 0,02 % à 5 % en poids par rapport au poids de la composition.

Comme pigments minéraux utilisables dans l'invention, on peut citer les oxydes de titane, de zirconium ou de cérium ainsi que les oxydes de zinc, de fer ou de chrome, le bleu ferrique, le violet de manganèse, le bleu outremer et l'hydrate de chrome. Parmi les pigments organiques utilisables dans l'invention, on peut citer le noir de carbone, les pigments de type D & C, et les laques à base de carmin de cochenille, de baryum, strontium, calcium, aluminium ou encore les dicéto pyrrolopyrrole (DPP) décrits dans les documents EP-A-542669, EP-A-787730, EP-A-787731 et WO-A- 96/08537. La quantité et/ou le choix de ces pigments sont généralement ajustés en prenant en compte la quantité en nanotubes présente dans la composition cosmétique considérée.

Les nacres peuvent être présentes dans la composition à raison de 0,01 % à 25 % en poids, notamment de 0,01 % à 15 % en poids, et en particulier de 0,02 % à 5 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition peut comprendre également des colorants hydrosolubles ou liposolubles en une teneur allant de 0,01 % à 6 % en poids, par rapport au poids total de la composition, notamment allant de 0,01 % à 3 % en poids. Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, et le jaune quinoléine. Les colorants hydrosolubles sont par exemple le jus de betterave et le bleu de méthylène.

La composition selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 % à 20 % du poids total de la composition.

Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction considérée.

La composition de l'invention peut être obtenue selon les procédés de préparation classiquement utilisés en cosmétique ou en dermatologie.

La composition de l'invention peut se présenter sous la forme de composition solide, compactée ou coulée en stick ou en coupelle, pâteuse ou liquide. Avantageusement, elle se présente sous forme solide, à savoir sous forme dure (ne s'écoulant pas sous son propre poids) notamment coulée ou compactée, par exemple en stick ou en coupelle.

En l'occurrence, elle peut se présenter sous la forme de rouges à lèvres, baumes à lèvres, fonds de teint coulés, produits anti-cernes, produits « correcteurs » ou « embellisseurs » de teint, et/ou fards à paupières ou à joues.

Elle peut toutefois se présenter sous forme de pâte, de solide ou de crème. Elle peut être une émulsion huile-dans-eau ou eau-dans-huile, un gel anhydre, solide ou souple ou encore sous forme de poudre libre ou compactée et même sous forme biphasique. Selon une variante particulière, elle se présente sous la forme d'une émulsion.

La composition selon l'invention peut se présenter sous la forme d'une composition, colorée ou non, sous forme d'une composition de protection solaire ou de démaquillage ou encore sous forme d'une composition hygiénique. Elle contient notamment des actifs cosmétiques. Elle peut alors être utilisée comme base de soin ou de traitement pour la peau comme les mains ou le visage ou pour les lèvres (baumes à lèvres, protégeant les lèvres du froid et/ou du soleil et/ou du vent) ou encore comme déodorant. Comme actif cosmétique utilisable dans l'invention, on peut citer les vitamines A, E, C, B3, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao, diméthicone), les hydratants (arginine PCA), les actifs antirides, les acides gras essentiels, et leurs mélanges.

La composition de l'invention peut également se présenter sous la forme d'un produit de maquillage de la peau, en particulier du visage comme un fond de teint, un blush, un fard comme un produit de tatouage semi-permanent ou de maquillage des lèvres comme un rouge ou un brillant à lèvres, présentant éventuellement des propriétés de soin ou de traitement, un produit de maquillage des phanères comme par exemple un vernis à ongles, un mascara, un eyeliner, un produit de coloration ou de soin des cheveux.

Bien entendu la composition de l'invention doit être cosmétiquement acceptable, à savoir non toxique et susceptible d'être appliquée sur la peau, les phanères ou les lèvres d'êtres humains.

Les exemples de compositions ci-après sont donnés à titre illustratif et sans caractère limitatif.

### EXEMPLE 1

Deux formulations gloss sont préparées :
- l'une, conforme à l'invention, incorpore l'ester de diacide dilinoléique et de diol dilinoléique commercialisé sous la dénomination LUSPLAN DD-DA7® , et
- l'autre, dite comparative, remplace ledit ester à quantité équivalente par un polybutylène commercialisé sous la dénomination INDOPOL H-1500® par la société AMOCO.

Leurs compositions respectives sont les suivantes :

| Phase | Matières premières | Essai 1 (Invention) | Essai 2 (comparatif) |
|---|---|---|---|
| A- | Malate de di-iso-stéaryle | 50,9 | 50,9 |
| | Ester d'acides gras végétaux, iso-stearique, adipique de glycéryle (Softisan 649® ) | 9,5 | 9,5 |
| | Conservateur | qs | qs |
| | Mélange de p-hydroxybenzoates d'iso-propyle, iso-butyle, n-butyle | 0,29 | 0,9 |
| B- | Polybutène (INDOPOL H-1500® d'AMOCO) | - | 30 |
| | Copolymères de diacides dilinoléiques et de diols dilinoléiques (LUSPLAN DD-DA7 de NIPPON FINE CHEMICAL) | 30 | - |
| C- | Pigment | 2 | 2 |
| D- | Silice | 0,3 | 0,3 |
| E- | Parfum | 7 | 7 |

Le protocole de préparation est comme suit :

Le pigment (phase C) est broyé dans 30g de phase A. Le broyat, le reste de la phase A et la phase B, sont ensuite ajoutés dans un poëlon chauffé à 70 °C et le tout est homogénéïsé pendant 30 minutes à l'aide d'un raynerie. On ajoute ensuite la silice (phase D) au mélange et on homogénéïse pendant 2 heures. Enfin, le parfum est ajouté au mélange. On homogénéïse pendant 5 minutes et on stoppe l'agitation et le chauffage. On laisse refroidir à température ambiante et on conditionne la pâte souple obtenue dans les tubes.

On note que la formule contenant l'ester diacide dilinoléique / diol dilinoléique présente de meilleures propriétés cosmétiques que celles contenant les polybutylènes. Ainsi, la formule avec l'ester diacide dilinoléique / diol dilinoléique LUSPLAN DD-DA7® est moins collante et a tendance à être plus glissante et plus brillante à l'application que celle contenant le polybutylène INDOPOL H-1500® . Elle est, de plus, moins collante et plus brillante après application. Au bout d'une heure, elle est moins collante et a tendance à être plus brillante.

### EXEMPLE 2

Les deux formulations précédentes ont été reproduites en remplaçant dans l'essai 1 le LUSPLAN DD-DA7® par le LUSPLAN DD-DA5® et l'INDOPOL H-1500® de l'essai 2 par l'INDOPOL H-100.

On note que la formule conforme à l'invention est plus brillante au terme d'une heure que la formulation témoin.

### EXEMPLE 3

Deux formulations stick sont préparées :
- l'une, conforme à l'invention, incorpore l'ester de diacide dilinoléique et de diol dilinoléique commercialisé sous la dénomination LUSPLAN DD-DA5® , et
- l'autre, dite comparative, remplace ledit ester à quantité équivalente de lanoline liquide.

Leurs compositions respectives sont les suivantes :

| Phase | Matières premières | Essai 3 (Invention) | Essai 4 (comparatif) |
|---|---|---|---|
| A- | Huile de sésame | 24 | 24 |
| | Conservateur | qs | qs |
| | Copolymères diacides dilinoléiques/diols dilinoléiques (LUSPLAN DD-DA5® de Nippon Fine Chemical) | 16,1 | - |
| | Lanoline liquide | - | 16,1 |
| | Esters d'acides gras végétaux, isostéarique, adipique de glycéryle | 8 | 8 |
| B- | Huile de sésame | 12,1 | 12,1 |
| | Hectorite modifiée par le chlorure de di-stéaryl diméthyl ammonium | 0,6 | 0,6 |
| | Copolymères dimères dilinoléyl diol / dimères dilinoléiques (LUSPLAN DD-DA5® ) | 8,1 | 8,1 |
| | Lanoline liquide | - | - |
| | Esters d'acides gras végétaux, isostéarique, adipique de glycéryle (Softisan 649®) | 4,1 | 4,1 |
| C- | Cire microcristalline (MICROWAX HW® de Paramelt) | 2,5 | 2,5 |
| | Cire d'abeille polyglycérolée | 4,2 | 4,2 |
| | Cire microcristalline (BASE WAX 30540® de Paramelt) | 8 | 8 |
| D- | Dioxide de titane | 1,8 | 1,8 |
| | Pigments | 6,9 | 6,9 |
| E- | Actifs | 3,6 | 3,6 |

Le protocole de préparation est comme suit :

Le gel de bentone (phase B) est réalisé à l'aide d'un homogénéisateur haute pression. Les pigments (phase D) sont broyés dans la phase A. Puis le tout est ajouté dans un poêlon avec la phase C, chauffé à 100 °C et homogénéisé à l'aide d'un raynerie pendant deux heures. 5 minutes avant le coulage, on ajoute la phase active E. On coule ensuite l'ensemble dans un moule à 42 °C et on met les moules au congélateur à -20 °C pendant une demi-heure avant de procéder au démoulage des sticks.

On note que, appliquée sur des lèvres, la formule contenant l'ester diacide dilinoléique / diol dilinoléique est plus brillante à 1 heure que celle contenant la lanoline liquide, toutes les autres propriétés étant identiques par ailleurs.

## Revendications

1. Composition cosmétique de maquillage et/ou de soin de la peau, des lèvres et/ou des phanères comprenant dans un milieu physiologiquement acceptable au moins un ester de dimère diol et d'au moins un acide mono-carboxylique en C₄ à C₃₄ ou dicarboxylique, au moins un composé pâteux et au moins un agent de coloration, ladite composition possédant une brillance moyenne supérieure ou égale à 30.

2. Composition cosmétique anhydre de maquillage et/ou de soin de la peau, des lèvres et/des phanères comprenant dans un milieu physiologiquement acceptable au moins un ester de dimère diol et d'acide gras ou de dimère diacide d'acide gras, au moins un composé pâteux et moins de 15 % en poids d'au moins une charge.

3. Composition cosmétique de maquillage et/ou de soin de la peau, des lèvres et/ou des phanères comprenant dans un milieu physiologiquement acceptable au moins un ester de dimère diol et de dimère diacide d'acides gras insaturés et au moins un composé pâteux.

4. Composition selon la revendication 1, **caractérisée en ce que** l'acide mono-carboxylique est choisi parmi
- les acides linéaires saturés tels que l'acide butanoïque, l'acide pentanoïque, l'acide hexanoïque, l'acide heptanoïque, l'acide octanoïque, l'acide nonanoïque, l'acide décanoïque, l'acide undécanoïque, l'acide dodécanoïque, l'acide tridécanoïque, l'acide tétradécanoïque, l'acide heptadécanoïque, l'acide hexadécanoïque, l'acide pentadécanoïque, l'acide octadécanoïque, l'acide nonadécanoïque, l'acide eicosanoïque, l'acide docosanoïque, l'acide tétracosanoïque,
- les acides gras,
- des hydroxyacides tels que l'acide 2-hydroxybutanoïque, l'acide 2-hydropentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytridécanoïque, l'acide 2-hydroxytétradécanoïque, l'acide 2-hydroxyhexadécanoïque, l'acide 2-hydroxyheptadécanoïque, l'acide 2-hydroxyoctadécanoïque, l'acide 12-hydroxyoctadécanoïque, l'acide 2-hydroxynonadécanoïque, l'acide 2-hydroxyeicosanoïque, l'acide 2-hydroxydocosanoïque, l'acide 2-hydroxytétracosanoïque et
- des acides cycliques tels que l'acide cyclohexanoïque, la rosine hydrogénée, la rosine, l'acide abiétique, l'acide abiétique hydrogéné, l'acide benzoïque, l'acide p-oxybenzoïque, l'acide p-aminobenzoïque, l'acide cinnamique, l'acide p-méthoxycinnamique, l'acide salicylique, l'acide gallique, l'acide pyrrolidonecarboxylique, l'acide nicotinique et leurs mélanges.

5. Composition selon l'une quelconque des revendications 2 et 4, **caractérisée en ce que** l'acide gras est choisi parmi :
- des acides gras ramifiés tels que l'acide isobutanoïque, l'acide isopentanoïque, l'acide pivalique, l'acide isohexanoïque, l'acide isoheptanoïque, l'acide isooctanoïque, l'acide diméthyloctanoïque, l'acide isononanoïque, l'acide isodécanoïque, l'acide isoundécanoïque, l'acide isododécanoïque, l'acide isotridécanoïque, l'acide isotétradécanoïque, l'acide isopentadécanoïque, l'acide isohexadécanoïque, l'acide isoheptadécanoïque, l'acide isooctadécanoïque, l'acide isononadécanoïque, l'acide isoeicosanoïque, l'acide 2-éthylhexanoïque, l'acide 2-butyloctanoïque, l'acide 2-hexyldécanoïque, l'acide 2-octyldodécanoïque, l'acide 2-décyltétradécanoïque, l'acide 2-dodécylhexadécanoïque, l'acide 2-tétradécyloctadécanoïque, l'acide 2-hexadécyloctadécanoïque,
- des acides gras linéaires insaturés en C₈ à C₃₄, tels que l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et
- des acides gras d'origine naturelle, tels que les acides gras d'huile d'orange, d'huile d'avocat, d'huile de macadamia, d'huile d'olive, d'huile de soja hydrogénée, d'huile de jojoba, d'huile de palme, d'huile de ricin, d'huile de germe de blé, d'huile de safran, d'huile de grains de coton, d'huile de vison et leurs mélanges.

6. Composition selon l'une quelconque des revendications 1 et 4 à 5, **caractérisée en ce que** l'acide dicarboxylique est choisi parmi :
- les composés de formule (I) suivante :
HOOC-(CH₂)ₙ-COOH (I)
dans laquelle n est un nombre entier de 1 à 16, de préférence de 3 à 16, et
- les dimères diacides obtenus par dimérisation intermoléculaire d'au moins un acide gras insaturé.

7. Composition selon la revendication 6, **caractérisée en ce qu'**il s'agit d'un dimère diacide.

8. Composition selon l'une quelconque des revendications 3 et 5 à 7, **caractérisée en ce que** l'acide gras insaturé est un acide gras insaturé en C₈ à C₃₄, notamment en C₁₂ à C₂₂, en particulier en C₁₆ à C₂₀ et plus particulièrement en C₁₈.

9. Composition selon l'une quelconque des revendications 3 et 6 à 8, **caractérisée en ce que** l'acide gras insaturé est choisi parmi l'acide undécénoïque, l'acide lindérique, l'acide myristoléique, l'acide palmitoléique, l'acide oléique, l'acide linoléique, l'acide élaïdinique, l'acide gadolénoïque, l'acide eicosapentaénoïque, l'acide docosahexaénoïque, l'acide érucique, l'acide brassidique, l'acide arachidonique et leurs mélanges.

10. Composition selon l'une quelconque des revendications 2, 3 et 6, **caractérisée en ce que** le dimère diacide dérive de la dimérisation de l'acide linoléique.

11. Composition selon l'une quelconque des revendications précédentes **caractérisée en ce que** le dimère diacide est sous sa forme hydrogénée.

12. Composition selon l'une quelconque des revendications précédentes, **caractérisée en ce que** le dimère diol dérive de l'hydrogénation d'un dimère diacide.

13. Composition selon la revendication 12, **caractérisée en ce que** le dimère diacide dérive de la dimérisation d'un acide gras insaturé, notamment en C₈ à C₃₄ et en particulier en C₁₂ à C₂₂, en particulier en C₁₆ à C₂₀, et plus particulièrement en C₁₈.

14. Composition selon la revendication précédente, **caractérisée en ce que** l'acide gras insaturé est tel que défini en revendication 5 ou 9.

15. Composition selon l'une quelconque des revendications précédentes, **caractérisée en ce que** le dimère diol dérive de l'hydrogénation du diacide dilinoléique.

16. Composition selon l'une quelconque des revendications précédentes, **caractérisée en ce que** le dimère diol est sous une forme saturée.

17. Composition selon l'une quelconque des revendications 12 à 16, **caractérisée en ce que** le dimère diacide est identique au dimère diacide dont dérive le dimère diol.

18. Composition selon l'une quelconque des revendications précédentes, **caractérisée en ce que** l'ester de dimère diol est un ester de dimère diol et de dimère diacide.

19. Composition selon l'une quelconque des revendications précédentes,
**caractérisée en ce que** l'ester est un composé de formule générale (II) :
HO-R¹-(-OCO-R²-COO-R¹-)ₕ-OH
dans laquelle :
R¹ représente un reste de dimère diol obtenu par hydrogénation du diacide dilinoléique,
R² représente un reste de diacide dilinoléique hydrogéné, et
h représente un entier variant de 1 à 9.

20. Composition selon l'une quelconque des revendications précédentes, **caractérisée en ce que** l'ester est présent à raison de 1 à 99 % en poids, notamment de 2 à 60 % en poids, en particulier de 5 à 40 % en poids et plus particulièrement de 10 à 35 % en poids par rapport au poids total de la composition.

21. Composition selon l'une quelconque des revendications précédentes, **caractérisée en ce qu'**elle présente une brillance moyenne supérieure ou égale à 30, notamment supérieure ou égale à 60 et en particulier supérieure ou égale à 70.

22. Composition selon l'une quelconque des revendications précédentes, **caractérisée en ce que** le composé pâteux possède une dureté à 20°C allant de 0,001 à 0,5 MPa, notamment de 0,002 à 0,4 MPa.

23. Composition selon l'une quelconque des revendications précédentes, **caractérisée en ce que** le composé pâteux présente à l'état solide une organisation cristalline anisotrope, et comporte à la température de 23°C une fraction liquide et une fraction solide.

24. Composition selon la revendication 23, **caractérisée en ce que** la fraction liquide à 23°C représente notamment entre 15 et 85 %, en particulier entre 40 et 85 % en poids.

25. Composition selon la revendication 23 ou 24, **caractérisée en ce que** la fraction liquide du composé pâteux mesurée à 32°C représente notamment de 30 à 100 % en poids du composé, en particulier de 80 à 100 %, plus particulièrement de 90 à 100 % en poids du composé.

26. Composition selon l'une quelconque des revendications précédentes, **caractérisée en ce que** le composé pâteux est choisi parmi :
- les composés siliconés polymères ou non,
- les composés fluorés polymères ou non,
- les polymères vinyliques, notamment:
- les homopolymères d'oléfines
- les copolymères d'oléfines
- les homopolymères et copolymères de diènes hydrogénés
- les oligomères linéaires ou ramifiés, homo ou copolymères de (méth)acrylates d'alkyles ayant de préférence un groupement alkyle en C₈-C₃₀
- les oligomères homo et copolymères d'esters vinyliques ayant des groupements alkyles en C₈-C₃₀
- les oligomères homo et copolymères de vinyléthers ayant des groupements alkyles en C₈-C₃₀,
- les polyéthers liposolubles résultant de la polyéthérification entre un ou plusieurs diols en C₂-C₁₀₀, de préférence en C₂-C₅₀,
- les esters,
et leurs mélanges.

27. Composition selon l'une quelconque des revendications précédentes, **caractérisée en ce que** le composé pâteux est hydrocarboné.

28. Composition selon l'une quelconque des revendications 1 à 26, **caractérisée en ce que** le composé pâteux est le polyméthyl trifluoropropyl méthylalkyl diméthylsiloxane.

29. Composition selon la revendication 26, **caractérisée en ce que** le polyéther liposoluble est choisi parmi les copolymères d'éthylène-oxyde et/ou de propylène-oxyde avec des alkylènes-oxydes à longue chaîne en C₆-C₃₀, en particulier encore tels que le rapport pondéral de l'éthylène-oxyde et/ou de propylène-oxyde avec alkylènes-oxydes dans le copolymère est de 5:95 à 70:30.

30. Composition selon la revendication 29, **caractérisée en ce que** le polyéther liposoluble est un copolymère bloc de polyoxyéthylène/polydodécyle glycol.

31. Composition selon la revendication 26, **caractérisée en ce que** les esters sont choisis parmi :
- les esters d'un glycérol oligomère, notamment les esters de diglycérol, en particulier les condensats d'acide adipique et de glycérol, pour lesquels une partie des groupes hydroxyles des glycérols ont réagi avec un mélange d'acides gras tels que l'acide stéarique, l'acide caprique, l'acide stéarique et l'acide isostéarique et l'acide 12-hydroxystéarique,
- le propionate d'arachidyle,
- les esters de phytostérol,
- les polyesters non réticulés résultant de la polycondensation entre un diacide ou un polyacide carboxylique linéaire ou ramifié en C₄-C₅₀ et un diol ou un polyol en C₂-C₅₀,
- les esters aliphatiques d'ester résultant de l'estérification d'un ester d'acide hydroxycarboxylique aliphatique par un acide carboxylique aliphatique; et leurs mélanges.

32. Composition selon la revendication précédente, **caractérisée en ce que** l'acide carboxylique est choisi parmi l'acide héxanoïque, l'acide heptanoïque, l'acide octanoïque, l'acide éthyl-2 héxanoïque, l'acide nonanoïque, l'acide décanoïque, l'acide undécanoïque, acide dodécanoïque, l'acide tridécanoïque, l'acide tétradécanoïque, l'acide pentadécanoïque, l'acide héxadécanoïque, l'acide héxyldécanoïque, l'acide heptadécanoïque, l'acide octadécanoïque, l'acide isostéarique, l'acide nonadécanoïque, l'acide eicosanoïque, l'acide isoarachidique, l'acide octyldodécanoïque, l'acide henéicosanoïque, l'acide docosanoïque, et leurs mélanges.

33. Composition selon la revendication 31, **caractérisée en ce que** l'ester d'acide hydroxycarboxylique aliphatique est choisi parmi :
a) les esters partiels ou totaux de monoacides carboxyliques aliphatiques mono hydroxylés linéaires, saturés ;
b) les esters partiels ou totaux de monoacides carboxyliques aliphatiques mono hydroxylés insaturés ;
c) les esters partiels ou totaux de polyacides carboxyliques aliphatiques mono hydroxylés saturés;
d) les esters partiels ou totaux de polyacides carboxyliques aliphatiques poly hydroxylés saturés ;
e) les esters partiels ou totaux de polyols aliphatiques en C₂ à C₁₆ ayant réagi avec un mono ou un poly acide carboxylique aliphatique mono ou poly hydroxylé,
et leurs mélanges.

34. Composition selon l'une quelconque des revendications précédentes, **caractérisée en ce que** le composé pâteux est présent à raison de 1 à 99 % en poids, notamment de 1 à 60 % en poids, en particulier de 2 à 30 % en poids et plus particulièrement de 5 à 15 % en poids par rapport au poids total de la composition.

35. Composition selon l'une quelconque des revendications précédentes, **caractérisée en ce qu'**elle possède un point de fusion ou une température de transition thermique supérieure à 25°C, notamment variant de 25 à 85 °C et en particulier variant de 30 à 60 °C et/ou une dureté allant de 0,001 à 0,5MPa, notamment allant de 0,005 à 0,4MPa.

36. Composition selon l'une quelconque des revendications 3 à 35, **caractérisée en ce qu'**elle comprend au moins une phase aqueuse.

37. Composition selon l'une quelconque des revendications précédentes, **caractérisée en ce qu'**elle comprend au moins une phase grasse.

38. Composition selon l'une quelconque des revendications 1 à 35 et 37, **caractérisée en ce qu'**elle est anhydre.

39. Composition selon la revendication 37 ou 38, **caractérisée en ce que** ladite phase grasse contient au moins un corps gras liquide à température ambiante et à pression atmosphérique et/ou au moins un corps gras solide à température ambiante et à pression atmosphérique.

40. Composition selon la revendication 39, **caractérisée en ce que** ledit corps gras liquide à température ambiante et à pression atmosphérique comprend au moins une huile volatile ou non volatile ou un de leurs mélanges.

41. Composition selon la revendication 40, **caractérisée en ce que** l'huile non volatile est choisie parmi les huiles hydrocarbonées d'origine animale ; les huiles hydrocarbonées végétales ; les hydrocarbures linéaires ou ramifiés d'origine minérale ou synthétique ; les éthers de synthèse ayant de 10 à 40 atomes de carbone; les esters de synthèse, les esters de polyol ; les alcools gras ayant de 12 à 26 atomes de carbone ; les acides gras supérieurs ; les huiles siliconées de type polyméthylsiloxanes (PDMS) et leurs mélanges.

42. Composition selon l'une quelconque des revendications 39 à 41, **caractérisée en ce que** le corps gras liquide comprend au moins une huile volatile choisie parmi les huiles hydrocarbonées ayant de 8 à 16 atomes de carbone et les huiles de silicones volatiles.

43. Composition selon l'une quelconque des revendications 39 à 42, **caractérisée en ce que** ledit corps gras liquide à température ambiante et à pression atmosphérique représente de 0,01 à 90 %, notamment de 0,1 à 85 %, en poids par rapport au poids total de la phase grasse.

44. Composition selon l'une quelconque des revendications 39 à 43, **caractérisée en ce que** ledit corps gras solide à température ambiante et à pression atmosphérique est choisi parmi les cires, les gommes et leurs mélanges.

45. Composition selon l'une quelconque des revendications 37 à 44, **caractérisée en ce que** ladite phase contient au moins un corps gras solide à raison de 0,01 à 50 %, notamment de 0,1 à 40 %, et en particulier de 0,2 à 30 %, en poids par rapport au poids total de la phase grasse.

46. Composition selon l'une quelconque des revendications précédentes, **caractérisée en ce qu'**elle comprend moins de 15% en poids, en particulier moins de 10% en poids et notamment moins de 7% en poids d'au moins une charge par rapport à son poids total.

47. Composition selon la revendication 46, **caractérisée en ce que** la charge est choisie parmi les charges sphériques comme le talc, le stéarate de zinc, le mica, le kaolin, les poudres de polyamide, les poudres de polyéthylène, les poudres de polymères de tétrafluoroéthylène, l'amidon, le nitrure de bore, des microsphères polymériques telles que celles de chlorure de polyvinylidène/acrylonitrile, de copolymères d'acide acrylique, les microbilles de résine de silicone, et les organopolysiloxanes élastomères.

48. Composition selon l'une quelconque des revendications précédentes, **caractérisée en ce que** ladite composition comprend en outre au moins un agent de coloration et notamment à raison de 0,01 à 40 %, notamment de 0,1 à 30 %, et en particulier de 0,2 à 25 %, en poids par rapport au poids total de ladite composition.

49. Composition selon la revendication 48, **caractérisée en ce que** ledit agent de coloration est choisi parmi les pigments, les colorants liposolubles ou hydrosolubles, les nacres et leurs mélanges.

50. Composition selon l'une quelconque des revendications 1 et 3 à 37 et 39 à 49, **caractérisée en ce qu'**elle se présente sous la forme d'une émulsion.

51. Composition selon l'une quelconque des revendications précédentes, **caractérisée en ce qu'**elle se présente sous la forme de produits coulés en stick ou en coupelle.

52. Composition selon l'une quelconque des revendications précédentes, **caractérisée en ce qu'**elle se présente sous la forme de rouges à lèvres, baumes à lèvres, fonds de teint coulés, produits anti-cernes, produits « correcteurs » ou « embellisseurs » de teint, et/ou fards à paupières ou à joues.

53. Procédé de maquillage et/ou de soin de la peau, des lèvres comprenant l'application sur la peau, les lèvres et/ou les phanères d'au moins une composition selon l'une quelconque des revendications 1 à 52.
